(19) Europäisches Patentamt
European Patent Office
Office européen des brevets

(11) Publication number: 0 466 966 A1

(12) **EUROPEAN PATENT APPLICATION**

(21) Application number: **90113948.5**

(22) Date of filing: **20.07.90**

(51) Int. Cl.5: **C08B 37/08**, C08H 1/00, A61K 37/02

Amended claims in accordance with Rule 86 (2) EPC.

(43) Date of publication of application: **22.01.92 Bulletin 92/04**

(84) Designated Contracting States: **AT BE CH DE DK ES FR GB GR IT LI LU NL SE**

(71) Applicant: **SEIKAGAKU CORPORATION**
**1-5, Nihonbashi-honcho 2-chome**
**Chuo-ku, Tokyo 103(JP)**

(72) Inventor: **Kimata, Koji**
**1404 Uedayama 1-chome, Tenpaku-ku**
**Nagoya-shi, Aichi(JP)**
Inventor: **Suzuki, Sakaru**
**Shiai-Manshon 2 Hongo A-1004, 167 Hongo 2-chome**
**Meito-ku, Nagoya-shi, Aichi(JP)**

(74) Representative: **Hansen, Bernd, Dr. Dipl.-Chem. et al**
**Hoffmann, Eitle & Partner Patent- und Rechtsanwälte Arabellastrasse 4 Postfach 81 04 20**
**W-8000 München 81(DE)**

(54) **Synthetic chondroitin sulfate proteoglycan and process for producing the same.**

(57) A synthetic chondroitin sulfate proteoglycan containing chondroitin sulfate and serum albumin chemically bound thereto, which inhibits the adhesion of various types of cells to fibronectin and related substances and a process for producing the same comprising binding chondroitin sulfate to serum albumin by a carbodiimide condensation method or a cyanogen bromide activation-method.

EP 0 466 966 A1

## FIELD OF THE INVENTION

This invention relates to a synthetic chondroitin sulfate proteoglycan containing chondroitin sulfate and serum albumin chemically bound thereto and a process for producing the same.

## BACKGROUND OF THE INVENTION

Chondroitin sulfate, which is isolated from cartilage, trachea, skin and other connective tissues, has been employed as a material for the production of drugs and medical materials.

However, the pharmacological effects of chondroitin sulfate which have been clinically proved are limited though it is expected to have some physiological activities based on its wide distribution *in vivo*.

## SUMMARY OF THE INVENTION

As a result of extensive studies on pharmacological effects of chondroitin sulfate, the present inventors found that chondroitin sulfate bound to serum albumin exerts pharmacological effects which have never been achieved by chondroitin sulfate *per se*.

An object of the present invention is to provide a synthetic chondroitin sulfate proteoglycan containing chondroitin sulfate and serum albumin chemically bound thereto and a process for producing the same.

## BRIEF DESCRIPTION OF THE DRAWING

Fig. 1 shows changes in adhesion of cells to immobilized fibronectin depending on the concentration of a glycosaminoglycan-binding serum albumin. In the figure, -●-shows the case of using synthetic CS-PG, -○- shows the case of using synthetic CS-PG2 and -△- shows the case of using synthetic CS-PG3.

## DETAILED DESCRIPTION OF THE INVENTION

Chondroitin sulfate to be used in the present invention is not particularly restricted. Examples thereof include chondroitin-4-sulfate (chondroitin sulfate A), chondroitin -6-sulfate (chondroitin sulfate C), chondroitin-4,6-disulfate, dermatan sulfate (chondroitin sulfate B), chondroitin sulfate D, chondroitin sulfate E, chondroitin sulfate K and chondroitin sulfate originating from sea-cucumber described in JP-A-63-10601 (the term "JP-A" as used herein means an "unexamined published Japanese patent application").

Usable as serum albumin are human serum albumin and bovine serum albumin. The serum albumin may be naturally occurring or synthetic, for example, genetically engineered ones.

The synthetic chondroitin sulfate proteoglycan of the present invention can be obtained by chemically binding the above-mentioned chondroitin sulfate to the serum albumin. More specifically, the proteoglycan of the present invention can be produced by covalently binding chondroitin sulfate to the serum albumin in such a manner as binding a carboxyl group to an amino group using a water-soluble carbodiimide; activating a carboxyl group and binding the resulting active ester to an amino group; activating a hydroxyl group with cyanogen halide and binding it to on amino group, a phosphate group or a hydroxyl group; converting an amino group into a bromoacetyl derivative, a diazonium derivative or a mercaptan derivative and binding it to an amino group, an imidazol group, a phenolic hydroxyl group, a phenyl group, a carboxyl group, a mercapto group or an aldehyde group; activating an hydroxyl group with a diepoxy or halogenated epoxy compound and binding it to an amino group or an hydroxyl group; and reacting a carboxyl group and an amino group with protonized Schiff base and subsequently with an isocyan compound (Ugi reaction) (Axen R. et al., Acta Chem. Scand., 25, 1129 (1971)). Among these, preferred are a carbodiimide condensation method and a cyanogen bromide activation method (Axen R. & Ernback S., Eur. J. Biochem., 18, 351 (1971)).

It is preferable that the synthetic chondroitin sulfate proteoglycan of the present invention is soluble in water in the case that it is applied to drugs.

In order to prepare such a water-soluble synthetic chondroitin sulfate proteoglycan, the content of the chondroitin sulfate in the proteoglycan may preferably range from 1.5 to 98 % by weight.

An amount of a condensation agent to be used in the binding reaction varies depending on the reaction method.

In the case of condensation using a water-solble carbodiimide, chondroitin sulfate and serum albumin, in an amount 0.02 to 10 times as much as the chondroitin sulfate, are dissolved in an aqueous solvent, the pH value of the resulting solution is adjusted to about 5 and the water-soluble carbodiimide is added to the

solution in an amount 0.05 to 5 times as much as the chondroitin sulfate at a temperature ranging from about 3 to 6 °C to obtain the synthetic chondroitin sulfate proteoglycan of the present invention.

Examples of the carbodiimide include diethyl carbodiimide, diisopropyl carbodiimide, methylpropyl carbodiimide, dicyclohexyl carbodiimide, hexamethylene carbodiimide, heptamethylene carbodiimide, 1-ethyl-3-(3-dimethylaminopropyl) carbodiimide, 1-cyclohexyl-3-(2-morpholinoethyl) carbodiimide meso-p-toluenesulfonate, 1-t-butyl-3-(3-dimethylaminopropyl) carbodiimide, diphenyl carbodiimide, 4,4'-dinitrodiphenyl carbodiimide, di-p-tolyl carbodiimide and bis(trimethylsilyl) carbodiimide.

A process for the synthesis of the chondroitin sulfate proteoglycan by the cyanogen bromide activation method is as follows. Chondroitin sulfate is dissolved in an aqueous solvent and activated with cyanogen bromide in an amount of 50% or less, preferably 5 to 20 % by weight of the chondroitin sulfate by a conventional manner. The activated chondroitin sulfate thus obtained is once precipitated with a hydrophobic solvent such as acetonitrile or tetrahydrofuran at a low temperature. After washing with the above-mentioned solvent, the precipitate is dissolved again in cold water. Then, a neutral buffer solution containing serum albumin in an amount 1 to 20 times as much as the chondroitin sulfate and the binding reaction is conducted. Thus, the desired synthetic chondroitin sulfate proteoglycan is obtained.

The synthetic chondroitin sulfate proteoglycan thus obtained can be purified by a conventional method such as dialysis, alcohol precipitation, gel filtration, ion exchange chromatography.

The synthetic chondrotin sulfate proteoglycan of the present invention shows an antagonic effect on fibronectin which is glycoprotein capable of promoting the adhesion and elongation of cells. Thus, it is expected to be useful as a tissue-adhesion inhibitor which may be topically applied to a patient for prevention of abdominal adhesion occurred after surgical operation. Neither chondroitin sulfate alone nor serum albumin alone shows such an effect.

To further illustrate the present invention, and not by way of limitation, the following Examples and Test Examples are given.

## EXAMPLE 1

Production of synthetic chondroitin sulfate proteoglycan by water-soluble carbodiimide condensation method

Chondroitin sulfate originating from whale cartilage (type A), chondroitin sulfate originating from shark cartilage (type C) and dermatan sulfate originating from swine skin (all mfd. by Seikagaku Corporation) and bovine serum albumin (Sigma Chemical Co.) were used.

5 mg of each of the above-mentioned glycosaminoglycans and 100 µg of bovine serum albumin (hereinafter referred to as "BSA") were dissolved in 0.5 ml of distilled water and the pH value of the resulting solution was adjusted to 5.0 with 0.1 M hydrochloric acid. To the solution was added 0.5 ml of distilled water (pH 5.0) containing 25 mg of 1-ethyl-3-(3-dimethylaminopropyl) carbodiimide hydrochloride. The mixture was stirred at 4 °C for 12 hours. Then, the mixture was centrifuged at 20,000 x g for 10 minutes and the supernatant was collected. After completely dialyzing the supernatant against distilled water. Then, the nondiffusible fraction containing the glycosaminoglycan-binding serum albumin was lyophilized to obtain the synthetic chondroitin sulfate proteoglycans. The thus-obtained products containing chondroitin sulfate (hereinafter referred to as "CS") (type A), CS (type C) and dermatan sulfate (hereinafter referred to as "DS") are designated as [synthetic CS-PG1], [synthetic CS-PG2] and [synthetic CS-PG3], respectively.

The physical properties of the products were as follows:

[synthetic CS-PG1]:
CS/BSA weight ratio: 14.3 (CS 93.5 %, BSA 6.5 %)
Sulfur content: 6.42 %
$[\alpha]_D$: - 29 (C = 1, $H_2O$)
[synthetic CS-PG2]:
CS/BSA weight ratio: 14.7 (CS 95.4 %, BSA 6.5 %)
Sulfur content: 6.59 %
$[\alpha]_D$: - 14 (C = 1, $H_2O$)
[synthetic CS-PG3]
CS/BSA weight ratio: 16.2 (CS 97.2 %, BSA 6.0 %)
Sulfur content: 6.54 %
$[\alpha]_D$: - 72.5 (C = 1, $H_2O$)

EXAMPLE 2

Production of synthetic chondroitin sulfate proteoglycan by water- soluble carbodiimide condensation method

100 mg of CS originating from bovine tracheal cartilage (molecular weight: 15,000 mfd. by Seikagaku Corporation) and 44 mg (0.67 µmole) or 4.4 mg (0.067 µmole) of BSA were each dissolved in 20 ml of distilled water and the pH value of the resulting solution was adjusted to 5.0 with 0.1 N hydrochloric acid. Then, 7.7 mg (40 µmole) of 1-ethyl-3-(3-dimethylaminopropyl)carbodiimide hydrochloride was added thereto. Each mixture thus obtained was stirred at 4 °C for 12 hours and centrifuged at 20,000 x g for 20 minutes. The supernatant was collected and dialyzed against distilled water. The nondiffusible fraction was then applied to a DEAE cellulofine column (1.5 x 5.6 cm) equilibrated with a 0.04 M tris hydrochloride buffer solution (pH 7.4) for adsorption. The column was washed with 100 ml of the same buffer to remove the unreacted BSA, and then, linear concentration gradient elution was carried out by using 200 ml portions of buffer solutions containing 0.1 M to 1.0 M sodium chloride. The fractions showing both peaks of protein and uronic acid were combined and subjedted to dialysis. The nondiffusible material in water was treated with ethanol to give a precipitate. The thus-obtained precipitate was washed with ethanol and dissolved in about 5 ml of distilled water. Then, the solution was lyophilized to obtain CS-BSA binding products. The product obtained by using 44 mg of BSA was designated as [synthetic CS-PG4] while the one obtained by using 4.4 mg of BSA was designated as [synthetic CS-PG5].

The physical properties of the products were as follows. [synthetic CS-PG4]:
Yield: 78.7 mg
CS/BSA weight ratio: 4.2 (CS 80.9 %, BSA 19.1 %)
Sulfur content: 6.38 %
$[\alpha]_D$: - 20 (C = 1, $H_2O$)
[synthetic CS-PG5]
Yield: 69.4 mg
CS/BSA weight ratio: 14.0 (CS 93.3 %, BSA 6.7 %)
Sulfur content: 6.77 %
$[\alpha]_D$: - 14 (C = 1, $H_2O$).

EXAMPLE 3

Production of synthetic chondroitin sulfate proteoglycan by water-soluble carbodiimide condensation method

The procedure of Example 1 was repeated except for using CS originating from shark cartilage (molecular weight: 30,000; type C) and CS originating from whale cartilage (molecular weight: 35,000; type A) and using each reactant in a weight ratio shown in Table 1. The product originating from the CS (type C) was designated as [synthetic CS-PG6] while the one originating from the CS (type A) was designated as [synthetic CS-PG7].

Table 1

| Product | Type of CS | Weight Ratio (mg) CS | BSA | WCC | Yield (mg) |
|---|---|---|---|---|---|
| [synthetic CS-PG6] | C | 100 | 10 | 2.9 | 92 |
| [synthitic CS-PG7] | A | 100 | 10 | 2.9 | 90 |

The physical properties of the products were as follows:
[synthetic CS-PG6]:
CS/BSA weight ratio: 10.0 (CS 90.9 %, BSA 9.1 %)
Sulfur content: 6.64 %
$[\alpha]_D$: - 15.6 (C = 1, $H_2O$)
[synthetic CS-PG7]:

CS/BSA weight ratio: 8.8 (CS 89.8 %, BSA 10.2 %)
Sulfur content: 6.62 %
$[\alpha]_D$: - 16.0 (C = 1, $H_2O$)

EXAMPLE 4

Production of synthetic chondroitin sulfate proteoglycan by cyanogen bromide activation method

1.0 g of CS originating from whale cartilage and 0.1 g of BSA were dissolved in 100 ml of a 0.1 M aqueous solution of sodium hydrogencarbonate. 0.2 g of cyanogen bromide was added thereto and the resulting mixture was allowed to react at 4 °C for 17 hours. Then, 0.1 ml of ethanolamine was added to the reaction mixture followed by the reaction at room temperature for 2 hours. After adjusting the pH value of the reaction mixture to 7.0 with 0.1 N hydrochloric acid, the mixture was dialyzed against running water for 2 hours. Then, ethanol was added to the nondiffusible fraction to give a precipitate and the precipitate thus formed was collected by centrifugation. The precipitate was washed with ethanol and dried to obtain the desired product as a white solid [synthetic CS-PG8]. The physical properties of the prodcut are as follows.
CS/BSA weight ratio: 10.3 (CS 92.1 %, BSA 8.9 %)
Sulfur content: 6.8 %
$[\alpha]_D$: - 15.5 (C = 1, $H_2O$)

EXAMPLE 5

Production of synthetic chondroitin sulfate proteoglycan by cyanogen bromide activation method

1.0 g of CS originating from shark cartilage was dissolved in a 5 M potassium phosphate buffer (pH 11.9) and cooled to 4 °C. Then, 2 ml of a solution of cyanogen bromide in acetonitrile (100 mg/ml) was added thereto and the thus-obtained mixture was allowed to react for 10 minutes. Then, acetonitrile 5 times as much as the reaction mixture was added to give a precipitate. The precipitate was thoroughly washed with acetonitrile and immediately thereafter it was dissolved in a 0.1 M solution of sodium hydrogencarbonate. A BSA aqueous solution (0.15 g/100 ml) was added thereto and the resulting mixture was allowed to react at 4°C for 17 hours. 0.1 ml of ethanolamine was then added to the reaction mixture followed by allowing to further react at room temperature for 2 hours. After adjusting the pH value of the reaction mixture to 7.0 with 0.1 N hydrochloric acid and ethanol was added thereto to give a precipitate. The precipitate was collected by centrifugation, washed with ethanol and dried to obtain the desired product as a white solid [synthetic CS-PG9]. The physical properties of the product are as follows.
CS/BSA weight ratio: 7.0 (CS 87.4 %, BSA 12.6 %)
Sulfur content: 6.6 %
$[\alpha]_D$: - 17 (C = 1, $H_2O$).

EXAMPLE 6

Production of synthetic chondroitin sulfate proteoglycan by water-soluble carbodiimide condensation method

50 mg of CS originating from swine skin and 22 mg of human serum albumin (hereinafter referred to as "HSA") were dissolved in 10 ml of distilled water and the pH value of the solution was adjusted to 5.0 with 0.1 N hydrochloric acid. Then, 7.7 mg (40 µmole) of 1-ethyl-3-(3-dimethylaminopropyl)carbodiimide hydrochloride was added thereto under ice-cooling. The resulting mixture was stirred at 4 °C for 12 hours and then centrifuged at 20,000 x g for 20 minutes. The supernatant was collected and dialyzed against distilled water. The nondiffusible material thus obtained was then applied to a DEAE cellulofine column (1.5 x 5.6 cm) equilibrated with a 0.04 M tris hydrochloride buffer solution (pH 7.4) for adsorption. After washing the column with 100 ml of same buffer to remove the unreacted BSA, linear concentration gradient elution was carried out using 200 ml portions of buffer solutions containing 0.1 M to 1.0 M sodium chloride. The fractions showing both peaks of protein and uronic acid were combined and dialyzed. Then, the nondiffusible material was lyophilized to obtain 35.9 mg of a CS (type B)-binding HSA product [synthetic CS-PG9]. The physical properties of the product are as follows.
CS/BSA weight ratio: 3.9 (CS 79.6 %, BSA 20.4 %)
Sulfur content: 6.2 %

$[\alpha]_D$: - 58 (C = 1, $H_2O$)

TEST EXAMPLE 1

Effect of synthetic chondroitin sulfate proteoglycan on adhesion of BHK cells to fibronectin

A 96-well incubation plate was coated with 5 µg/ml of human plasma fibronectin (available from Nitta Gelatin Inc.). Then, each synthetic chondroitin sulfate proteoglycan obtained in Example 1 was distributed to wells at the concentration specified in Fig. 1.

Separately, BHK cells (hamster newborn renal cells), which had been cultured to confluent, and 5 ml of trypsin at a concentration of 0.1 mg/ml were added to an incubation plate of 100 mm in diameter. After incubating at 37 °C for 5 minutes, 5 ml of a solution of soybean trypsin inhibitor (1 mg/ml) was added to cell culture to inactivate the trypsin. Then, the liberated cells were collected by centrifugation.

The cells were washed with a solution containing trypsin inhibitor twice and the cells were counted as a single cell suspension.

100 µl (1 x $10^4$ cells) of the single cell suspension thus obtained was added to the abovementioned incubation plate coated with the fibronectin and synthetic proteoglycan followed by incubation at 37 °C for 2 hours. After washing away the unadhered cells, the adhered cells were fixed with 2 % formaldehyde and stained with 1 % toluidine blue. Then, the number of cells were counted.

The results are shown in Fig. 1. Each value is a mean of data obtained by measuring twice. As shown in Fig. 1, it was found that the synthetic chondroitin sulfate proteoglycan of the present invention inhibited the cell-adhesion promoting effect of fibronectin. In a control case where an incubation plate coated with chondroitin sulfate C and 1-ethyl-3-(3-dimethylaminopropyl)carbodiimide was treated in the same manner, no cell adhesion was observed.

TEST EXAMPLE 2

Effect of synthetic chondroitin sulfate proteoglycan on adhesion of various cultured cells to immoblized fibronectin and related substances

(1) Effect on adhesion of BHK 21 cells to immobilized fibronectin and related substance

The synthetic chondroitin sulfate proteoglycans obtained in Example 2 were examined for inhibitory effect on adhesion of BHK 21 cells (newborn hamster renal cells) to fibronectin, laminin, I type collagen and vitronectin (all available from Nitta Gelatin Inc.). 5 µg/ml portions of human plasma fibronectin, laminin originating from mouse EHS tumor cells, I type collagen originating from rat tendon and bovine serum vitronectin were each applied to wells of a 96-well incubation plate. In the same manner as in Test Example 1, synthetic chondroitin sulfate (type A) proteoglycans were applied to each well. Then, 100 µl of a suspension of BHK 21 cells was added to each well and a change in cell-substrate adhesion was observed. A control plate which was treated with no synthetic chondroitin sulfate proteoglycan was also examined. The results are shown in Table 2 . In Table 2, relative cell adhesion number is semiquantitatively expressed, namely, "-" means that no or little cells adhered; "+" means that 10 to 20 % of the cells adhered; "+ + + +" means that 70 to 80 % of the cells adhered; and "+ + + + +" means that 90 to 100 % of the cells adhered.

Table 2

| Immobilized substance | Relative cell adhesion number/incubator (plate) Control | CS-PG4 | CS-PG5 |
|---|---|---|---|
| fibronectin | +++++ | - | - |
| laminin | + | - | - |
| collagen | + | - | - |
| vitronectin | ++++ | - | - |

(2) Effect on adhesion of CEF cell to immobilized fibronectin and related substances

The synthetic chondroitin sulfate proteoglycans obtained in Example 3 were examined for effects on adhesion of CEF cells (chick embryo fibroblasts) to human plasma fibronectin, EHS tumor mouse laminin, rat tendon I type collagen and bovine serum vitronectin in the same manner as in the above (1). The results are shown in Table 3.

Table 3

| Immobilized substance | Relative cell adhesion number/incubator (plate) Control | CS-PG6 | CS-PG7 |
|---|---|---|---|
| fibronectin | +++++ | - | - |
| laminin | + | - | - |
| collagen | + | - | - |
| vitronectin | ++++ | - | - |

(3) Effect on adhesion of CHO-K1 cell to immobilized fibronectin and related substance

The synthetic chondroitin sulfate proteoglycans obtained in Examples 4 and 5 were examined for effects on adhesion of CHO-K1 cells (Chinese hamster cells) to human plasma fibronectin and EHS tumor mouse laminin in the same manner as in the above (1). The results are shown in Table 4.

Table 4

| Immobilized substance | Relative cell adhesion number/incubator (plate) Control | CS-PG8 | CS-PG9 |
|---|---|---|---|
| fibronectin | +++++ | - | - |
| laminin | +++++ | - | - |

(4) Effect on adhesion of B16F10 cell to immobilized fibronectin and related substance

The synthetic chondroitin sulfate proteoglycan obtained in Example 6 was examined for adhesion of B16F10 cells (high-metastatic melanoma cells) to human plasma fibronectin and EHS tumor mouse laminin in the same manner as in the above (1). The results are shown in Table 5.

Table 5

| Cell adhesion substance | Relative cell adhesion number/incubator (plate) | |
|---|---|---|
| | Control | CS-PG10 |
| fibronectin | +++++ | - |
| laminin | +++ | - |

As shown in above results, it was found that the chondroitin sulfate proteoglycan according to the present invention inhibited the adhesion of various cells to fibronectin-like substance, particularly fibronectin.

**Claims**

1. A synthetic chondroitin sulfate proteoglycan containing chondroitin sulfate and serum albumin chemically bound thereto.

2. The synthetic chondroitin sulfate proteoglycan according to claim 1, wherein the chondroitin sulfate is covalently bound to the serum albumin.

3. The synthetic chondroitin sulfate proteoglycan according to claim 1, wherein the content of the chondroitin sulfate ranges from 1.5 to 98% by weight based on the chondroitin sulfate proteoglycan.

4. The synthetic chondroitin sulfate proteoglycan according to claim 1, wherein said serum albumin is human serum albumin or bovine serum albumin.

5. A process for producing a synthetic chondroitin sulfate proteoglycan containing chondroitin sulfate and serum albumin chemically bound thereto which comprises covalently binding chondroitin sulfate to serum albumin by a carbodiimide condensation method or a cyanogen bromides activation method.

6. Use of a synthetic chondroitin sulfate proteoglycan according to claim 1 for preparing a medicament for prevention of abdominal adhesion occurred after surgical operation.

Fig. 1

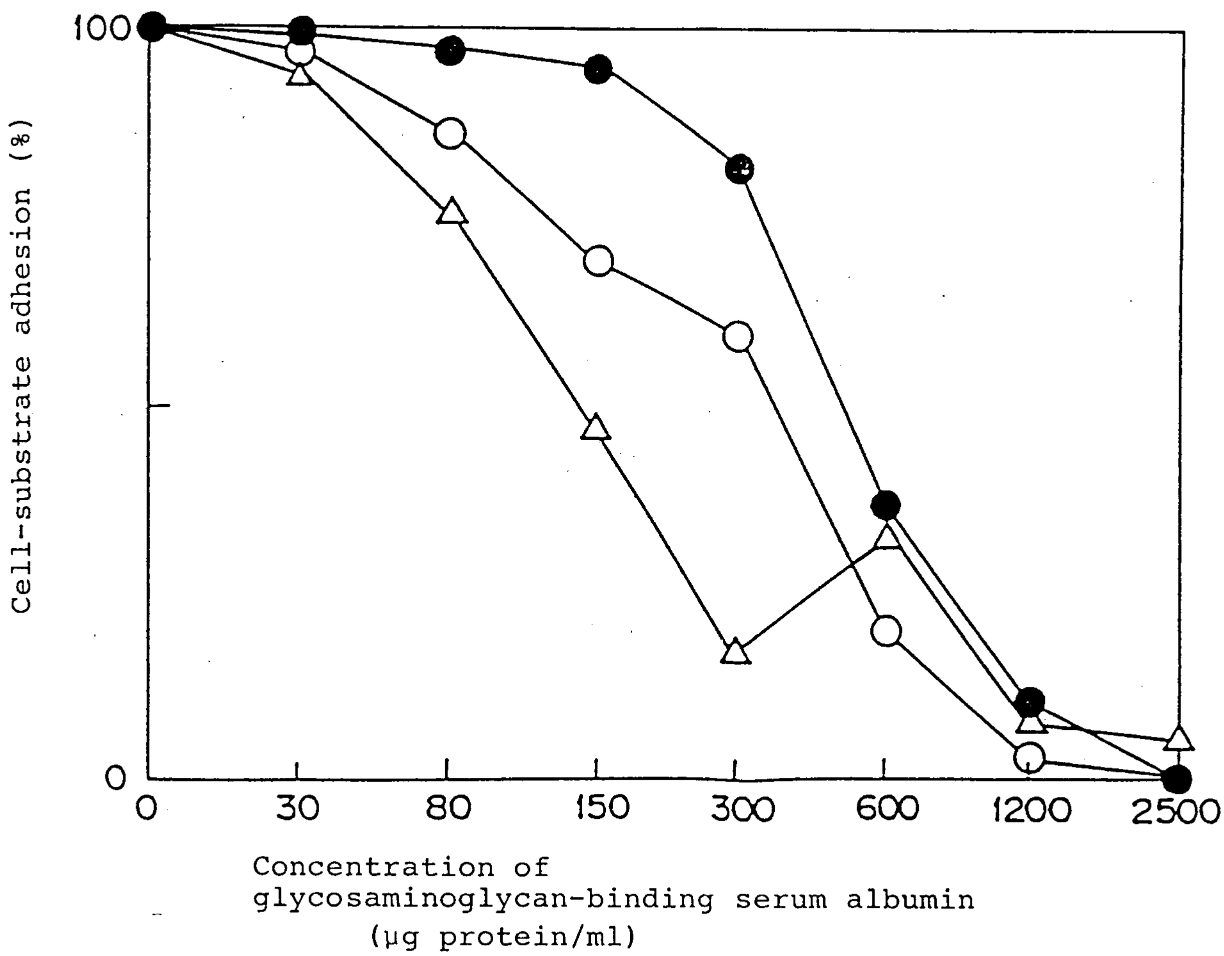
100
0
Cell-substrate adhesion (%)
0
30
80
150
300
600
1200
2500
Concentration of
glycosaminoglycan-binding serum albumin
(µg protein/ml)

European Patent Office

# EUROPEAN SEARCH REPORT

Application Number

**EP 90 11 3948**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | US-A-4 585 754 (MEISNER et al.) * Column 3, lines 43-68; claims * | 1-5 | C 08 B 37/08<br>C 08 H 1/00<br>A 61 K 37/02 |
| A | EP-A-0 081 853 (CORDIS EUROPA) | | |
| A | CARBOHYDRATE RESEARCH, vol. 111, no. 1, December 1982, pages 113-125; Y. INOUE et al.: "On the reaction of N-acetylchondrosine, N-acetylchondrosine 6-sulfate, chondroitin 6-sulfate, and heparin with 1-(3-dimethylaminopropyl)-3-ethylcarbodiimide" | | |
| A | US-A-4 657 820 (HALPERN et al.) * Example 3 * | 1 | |
| A | JAPANESE PATENTS GAZETTE, week J47, Section CH: Chemical , 1983, page 3, accession no. 01099 J, Derwent Publications Ltd, London, GB; & JP-A-57 167 919 (KYOSEI PHARMACEUTICAL) 16-10-1982 * Last paragraph * | 1 | |
| A | DERWENT JAPANESE PATENTS REPORT, vol. S, no. 14, 18th May 1971, accession no. 24118S, Derwent Publications Ltd, London, GB; & JP-B-71 13 265 (LION FAT AND OIL) | | **TECHNICAL FIELDS SEARCHED (Int. Cl.5)**<br>C 08 B<br>C 08 H |

The present search report has been drawn up for all claims

| Place of search | Date of completion of search | Examiner |
|---|---|---|
| The Hague | 27 March 91 | LENSEN H.W.M. |

CATEGORY OF CITED DOCUMENTS

X : particularly relevant if taken alone
Y : particularly relevant if combined with another document of the same catagory
A : technological background
O : non-written disclosure
P : intermediate document
T : theory or principle underlying the invention

E : earlier patent document, but published on, or after the filing date
D : document cited in the application
L : document cited for other reasons

& : member of the same patent family, corresponding document